# EUROPÄISCHE PATENTANMELDUNG

(11) **EP 1 279 732 A2**
(43) Veröffentlichungstag der Anmeldung: **29.01.2003**
(21) Anmeldenummer: 02090238.3
(22) Anmeldetag: 09.07.2002
(51) Int. Cl.: C12N 15/10

(54) **Verfahren zur Isolierung grosser Varianzen spezifischer Moleküle für ein Zielmolekül aus Phagemid-Gen-Bibliotheken**

(30) Priorität: 18.07.2001 DE 10135039
(71) Anmelder: Nemod Immuntherapie AG, 13125 Berlin (DE)
(72) Erfinder: Goletz, Steffen, 13129 Berlin (DE); Christensen, Peter Astrup, 10437 Berlin (DE); Kristensen, Peter, 8310 Tranbjerg J (DK)
(74) Vertreter: Ziebig, Marlene, Dr. Dipl.-Chem.

(57) **Zusammenfassung**

Die Erfindung betrifft ein Verfahren zur Isolierung großer Varianzen spezifischer Moleküle für ein Zielmolekül aus Phagemid-Gen-Bibliotheken und ein Testkit, indem von einer Phagemid-Gen-Bibliothek ausgegangen wird, die große Varianzen eines Moleküls oder große Varianzen von Teilen eines Moleküls, das im Sinne der Erfindung als spezifisches Molekül fungiert, direkt oder über einen Linker kovalent an das Phagenhüllprotein pIII des filamentösen Phagen M13 gekoppelt als Fusionsprotein auf der Oberfläche von Phagen enthalten, die Phagen der Bibliothek, die die Fusionsprotein exprimierenden Phagen enthalten, mit einem Zielmolekül inkubiert werden, die spezifisch über das Fusionsprotein an das Zielmolekül gebundenen Phagen mit einem Selektionsmolekül, das die Bindung zwischen dem spezifischen Molekül und dem Zielmolekül aufhebt und an das Zielmolekül bindet, eluiert werden, die eluierten Phagen, die das Fusionsprotein nicht exprimieren, derart inaktiviert werden, dass sie keine Bakterien mehr infizieren können, mit den eluierten Phagen, die das Fusionsprotein exprimieren, Baktieren infiziert werden und die Varianzen des spezifischen Moleküls aus den infizierten Klonen bestimmt und gewonnen werden.

## Beschreibung

Die Erfindung betrifft ein effizientes Verfahren zur Isolierung großer Varianzen spezifischer Moleküle für ein Zielmolekül aus Phagemid-Gen-Bibliotheken, das es erlaubt, große Varianzen spezifischer Moleküle, beispielsweise Antikörperfragmente wie Fab-Fragmente oder scFv-Fragmente, mit geringem Arbeitsaufwand zu isolieren. In einer besonderen Ausführungsform der Erfindung sind die zu isolierenden spezifischen Moleküle Surrogatmoleküle für das im erfindungsgemäßen Verfahren eingesetzte Selektionsmolekül.

Gegenstand der Erfindung ist auch ein Testkit zur Durchführung des erfindungsgemäßen Verfahrens.

Es ist bekannt, dass die Phagen-Display-Technik z.B. für die schnelle Herstellung spezifischer rekombinanter humaner Antikörperfragmente sehr gut geeignet ist. Um Fab-Fragmente oder single chain-Fv-Antikörperfragmente (scFv) mit einer gewünschten Spezifität herzustellen, werden diese aus Antikörper-Bibliotheken mit den entsprechenden Antigenen selektiert. Diese Antigene sind jedoch nicht immer in reiner Form verfügbar, sondern können z.B. Teil einer Antigen-Mischung sein. Selbst reine Antigene bestehen aus unterschiedlichen Epitopen, und Phagen-Display-Selektionen ergeben in den weitaus meisten Fällen üblicherweise Antikörper-Fragmente gegen ein oder wenige dominante(s) Epitop(e) des Antigens. Die Ursache dafür ist, dass normalerweise mehrere Selektionsrunden notwendig sind, um spezifische Antikörperfragmente anzureichern und zu isolieren. Am Ende werden die Antikörperfragmente, die mit der höchsten Affinität binden, oder die Phagen, die einen Wachstumsvorteil während der Amplifikation zwischen den Selektionsrunden haben, oder solche, die eine Kombination aus Wachstumsvorteil und hoher Affinität besitzen, erhalten. Antikörperfragmente mit geringeren Affinitäten gegen Epitope des Zielmoleküls oder solche mit einem Wachstumsnachteil im Phagen-Display System werden in der Regel nicht selektiert. Dieser Effekt wird größer mit zunehmender Zahl an Selektionsrunden. Dies führt zum Varianzverlust potentieller Binder während der sequentiellen Selektionsrunden und behindert die Selektion von Antikörpern geringerer Affinität oder gegen geringere Komponenten von Antigen-Mischungen oder Zelloberflächen.

Aus WO 99/58655 ist ein Selektionsverfahren bekannt, bei dem die Selektion der Phagen, die das gewünschte Fusionsprotein, wie beispielsweise das Antikörperfragment, an pIII gekoppelt exprimieren, unter Verwendung des Helferphagen KM13 durchgeführt wird, der ein modifiziertes pIII-Protein codiert, das eine proteolytische Spaltungsstelle aufweist. Dagegen kann der pIII-Teil des Fusionsproteins nicht proteolytisch gespalten werden, so dass die Infektiösität dieser Phagen, die das Fusionsprotein exprimieren, nach der proteolytischen Behandlung erhalten bleibt.

Diese Selektionsmethode erlaubt es, die Phagen, die das gewünschte Fusionsprotein nicht exprimieren und in Phagen-Display-Systemen oftmals einen starken Hintergrund darstellen, zu eliminieren. Mit Hilfe dieser Methode können einfacher spezifische Moleküle gewonnen werden. Allerdings lassen sich mit dieser Selektionsmethode keine großen Varianzen an spezifischen Molekülen wie z.B. Antikörperfragmenten isolieren.

Aufgabe der Erfindung war es, ein Selektionsverfahren zur Isolierung von spezifischen Molekülen aus Phagemid-Gen-Bibliotheken zur Verfügung zu stellen, das es erlaubt, die für ein Zielmolekül spezifischen Moleküle, insbesondere Antikörperfragmente, in großen Varianzen bereitzustellen. Insbesondere die Herstellung von Surrogatmolekülen wie beispielsweise scFv-Antikörperfragmenten mit der Standardmethode der Hybridomtechnik ist schwierig und langwierig, so dass mit dem Verfahren der Erfindung insbesondere solche Antikörperfragmente in effektiver Art und Weise bereitgestellt werden sollen.

Die Aufgabe der Erfindung wird gelöst, indem von einer Phagemid-Gen-Bibliothek ausgegangen wird, die große Varianzen eines Moleküls oder große Varianzen von Teilen eines Moleküls, das im Sinne der Erfindung als spezifisches Molekül fungiert, direkt oder über einen Linker kovalent an das Phagenhüllprotein pIII des filamentösen Phagen M13 gekoppelt als Fusionsprotein auf der Oberfläche von Phagen enthalten, die Phagen der Bibliothek, die die Fusionsprotein exprimierenden Phagen enthalten, mit einem Zielmolekül inkubiert werden, die spezifisch über das Fusionsprotein an das Zielmolekül gebundenen Phagen mit einem Selektionsmolekül, das die Bindung zwischen dem spezifischen Molekül und dem Zielmolekül aufhebt und an das Zielmolekül bindet, eluiert werden, die eluierten Phagen, die das Fusionsprotein nicht exprimieren, derart inaktiviert werden, dass sie keine Bakterien mehr infizieren können, mit den eluierten Phagen, die das Fusionsprotein exprimieren, Baktieren infiziert werden und die Varianzen des spezifischen Moleküls aus den infizierten Klonen bestimmt und gewonnen werden.

Es ist erfindungsgemäß auch möglich, dass die Inaktivierung der Infektiösität der Phagen, die kein spezifisches Molekül tragen, nicht als letzter Schritt, sondern bereits vor der Inkubation mit dem Zielmolekül und der spezifischen Elution vorgenommen wird.

Die zur Ausführung eingesetzten Phagemid-Gen-Bibliotheken des filamentösen Phagen M13, die große Varianzen der gewünschten Moleküle exprimieren, können nach an sich bekannten Methoden hergestellt werden (Nissim, A. *et al.* 1994, EMBO J. 13:692-69; Griffiths,A. et al., 1994, EMBO J., 13: 3245-3260; http://www.mrc-cpe.cam.ac.uk/∼phage/). Die Herstellung solcher Bibliotheken ist dem Fachmann bekannt.

In einer bevorzugten Ausführungsform der Erfindung werden als spezifische Moleküle Antikörperfragmente isoliert, bevorzugt single-domain Fragmente, Fab- oder scFv-Fragmente, oder Trägerproteine mit ein oder mehreren variablen Anteilen, vorzugsweise solche Moleküle die eine Grundstruktur besitzen, die es erlaubt, dass die variablen Anteile mit dem Zielmolekül spezifisch interagieren können, oder Peptide, die ganz oder teilweise variable Anteile haben. Unter Trägerproteinen versteht man dabei solche Proteinstrukturen, die als Rahmen/Grundstruktur einer Präsentation der variablen Anteile dienen, aber auch Proteinstrukturen im allgemeinen. Variable Anteile können innerhalb der Trägerproteinstruktur liegen, Teil der Trägerproteinstruktur sein oder an den Enden der Trägerproteine sitzen.

Im ersten Schritt des erfindungsgemäßen Verfahrens werden die Phagen, die die gewünschten spezifischen Moleküle tragen, in an sich üblicher Weise selektioniert, indem mit einem Zielmolekül inkubiert wird, wobei das Zielmolekül vorzugsweise immobilisiert an einer festen Phase, wie beispielsweise Magnetbeads oder Plastikoberflächen, eingesetzt wird (Phagen-Panning).

Im zweiten Schritt des erfindungsgemäßen Verfahrens werden dann die spezifisch über das Fusionsprotein an das Zielmolekül gebundenen Phagen mit einem Selektionsmolekül, das die Bindung zwischen dem spezifischen Molekül und dem Zielmolekül aufhebt (und an das Zielmolekül bindet), eluiert. Bevorzugt hebt das Selektionsmolekül die Bindung zwischen dem spezifischen Molekül und dem Zielmolekül auf, indem es z.B. kompetitiv oder allosterisch wirkt.

In einer bevorzugten Ausführungsform der Erfindung werden als Zielmoleküle Antikörper oder Antikörperfragmente verwendet, die ein Antigen spezifisch binden. Das Antigen oder geeignete Teile des Antigens werden als Selektionsmolekül eingesetzt, um große Varianzen spezifischer Moleküle zu gewinnen, die Surrogat-Moleküle für das Antigen darstellen. Das Antigen oder dessen Teile können als solche oder an Trägerstrukturen gekoppelt eingesetzt werden.

In einer anderen bevorzugten Ausführungsform der Erfindung werden als Zielmoleküle Rezeptoren oder Teile von Rezeporen verwendet, die einen Liganden spezifisch binden. Der Ligand oder geeignete Teile des Liganden werden als Selektionsmolekül eingesetzt, um große Varianzen spezifischer Moleküle zu gewinnen, die Surrogat-Moleküle für den Liganden darstellen. Die Liganden oder deren Teile können als solche oder an Trägerstrukturen gekoppelt eingesetzt werden.

In einer weiteren bevorzugten Ausführungsform der Erfindung werden als Zielmoleküle Moleküle biologischer oder nicht biologischer Herkunft verwendet, die keine Antikörper und auch keine Rezeptoren im oben erwähnten Sinne sind, jedoch ein Molekül oder mehrere Moleküle spezifisch binden, von dem oder denen Surrogat-Moleküle hergestellt werden sollen. Ein Beispiel hierfür sind Lektine. Moleküle oder entsprechende Teile von Molekülen, die das Zielmolekül binden und damit die spezifischen Moleküle eluieren, werden als Selektionsmolekül eingesetzt um große Varianzen spezifischer Moleküle zu gewinnen. Werden Lektine erfindungsgemäß als Zielmolekül eingesetzt, so dienen Kohlenhydratstrukturen oder deren Teile als Selektionsmolekül, wobei die Kohlenhydratstrukturen als solche oder an Träger gekoppelt verwendet werden können.

Im dritten Schritt des erfindungsgemäßen Verfahrens werden die eluierten Phagen, die das Fusionsprotein nicht exprimieren, derart inaktiviert, dass sie keine Bakterien mehr infizieren können. Wie oben beschrieben, könnte dieser Schritt auch bereits vor der Inkubation mit dem Zielmolekül vorgenommen werden, so dass er an dieser Stelle entfallen würde. Mit den eluierten Phagen, die das Fusionsprotein exprimieren, werden Bakterien infiziert. Vorzugsweise, werden die Bakterien, die durch einen Phagen infiziert wurden und damit das Gen für das entsprechende spezifische Molekül tragen, durch ein auf dem genetischen Material des Phagemid codierten Resistenzgen nach an sich bekannten Methoden auf Selektionsmedium selektiv gezüchtet. Entsprechende Klone werden dabei aus Kolonien gewonnen. Zur Analyse und weiteren Verwendung können die Sequenzen der spezifischen Moleküle nach herkömmlichen Methoden bestimmt und dabei die Varianzen bestimmt und die biologische Feincharakterisierung der spezifischen Moleküle in Form von in Bakterien exprimierten spezifischen Molekülen oder als Fusionsproteine auf Phagen, die mit Hilfe einer Superinfektion mit einem Helferphagen hergestellt werden, in geeigneten Testsystemen untersucht werden.

Die Inaktivierung der Phagen, die das spezifische Molekül nicht exprimieren, kann beispielsweise unter Verwendung eines Helferphagen erfolgen, der durch einen Inaktivierungsstoff inaktiviert wird. So kann beispielsweise ein Helferphage eingesetzt werden, der ein pIII trägt, das im Gegensatz zum pIII des Fusionsproteins proteolytisch spaltbar ist, z.B. durch Trypsin im Falle eines mit einer Trypsin-Spaltungsstelle im pIII modifizierten Helferphagen.

Gegebenenfalls werden die Schritte 1-3 in weiteren Selektionsrunden wiederholt durchgeführt. Hierfür werden die mit den eluierten Phagen infizierten Bakterien durch eine Superinfektion mit einem Helferphagen zur Produktion der Phagen mit den spezifischen Molekülen gebracht, wobei aufgrund des Phagemid Systems, wie bei der Gewinnung der Phagen der Gesamtbibliothek, ein Großteil der produzierten Phagen kein spezifisches Molekül tragen.

Wie bereits oben klargestellt, kann auch die Abfolge der beschriebenen Schritte derart verändert werden, dass der jetzt als Schritt 3 bezeichnete Schritt bereits vor dem jetzt als Schritt 1 bezeichneten Schritt vorgenommen wird, also vor der Inkubation mit dem Zielmolekül, der spezifischen Elution und den entsprechenden Waschschritten. Die Waschschritte, die von dem Zielmolekül, Selektionsmolekül und spezifischen Molekülen abhängen,sind nicht im Detail erläutert, da sie fachmännische Routine sind. Den Ausführungsbeispielen sind sie zu entnehmen.

Überraschenderweise können durch das erfindungsgemäße Verfahren große Sets an spezifischen Molekülen isoliert werden, die eine sehr hohe, bisher nicht erreichte Diversität zeigen und trotzdem ihre Spezifität für den Liganden erhalten haben. Dies wird erreicht durch die Kombination der spezifischen Elution mit Hilfe des Selektionsmoleküls, mit der Infektionsinaktivierung solcher Phagen, die unerwartet unspezifisch eluierten und keine spezifischen Moleküle tragen. Das Verfahren erlaubt die Isolation von einer großen Varianz spezifischer Moleküle bereits nach einer bis wenigen Selektionsrunden, wobei zwei oder drei Selektionsrunden bevorzugterweise verwendet werden.

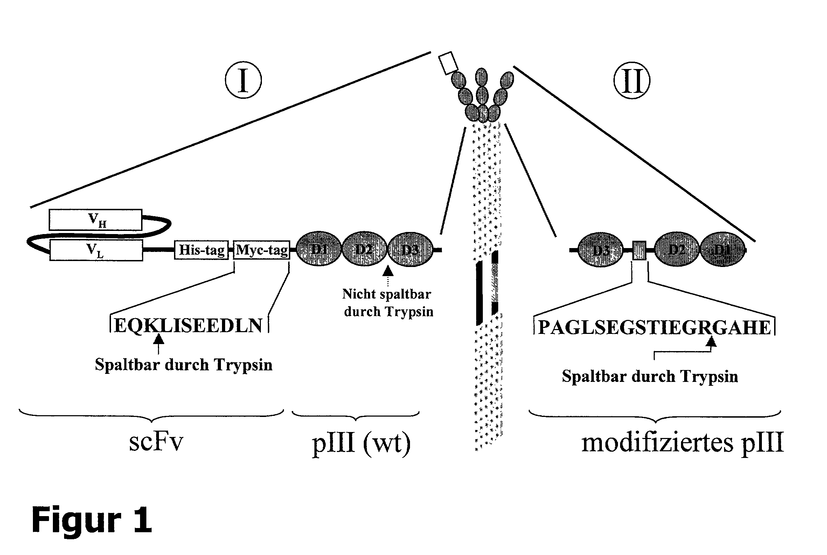

In einer bevorzugten Ausführungsform der Erfindung kann das Verfahren zur Isolierung großer Varianzen von Surrogat-Molekülen für ein tumorassoziiertes Antigen (z.B. Lewis Y) in der Form von Antikörperfragmenten, wie scFv-, single-domain- und Fab- Antikörperfragmenten, mit Hilfe von spezifischen Antikörpern dienen Dazu werden scFvpIII(Wildtyp)-Phagemidvektoren und modifizierte pIII-Helferphagen, die durch Einführung einer Trypsin-Spaltungsstelle proteolytisch spaltbar gemacht wurden, eingesetzt. Ein Beispiel ist in Figur 1 (vgl. auch Beispiel 1) dargestellt. Die Verwendung des proteolytisch spaltbaren Helferphagen in Kombination mit der Antigen-spezifischen Elution (z.B. mit dem Lewis Y Antigen) führte zu einer überraschend hohen Varianz an Surrogat-Molekülen in Form von scFv-Fragmenten, die die Bindung der Antikörper an das Antigen inhibieren.

In einer weiteren bevorzugten Ausführungsform der Erfindung kann das Verfahren zur Isolierung großer Varianzen von Surrogat-Molekülen in der Form von Antikörperfragmenten, wie scFv-, single-domain- und Fab- Antikörperfragmente, dienen, die gegen Rezeptoren (z.B. E-Selektin) gerichtet sind, und Surrogat-Moleküle für deren Liganden (z.B. Sialyl-Lewis A) darstellen. Dazu werden scFv-pIII(Wildtyp)-Phagemidvektoren und modifizierte pIII-Helferphagen, die durch Einführung einer Trypsin-Spaltungsstelle proteolytisch spaltbar gemacht wurden, eingesetzt. Ein Beispiel dafür ist in Ausführungsbeispiel 2 dargestellt. Die Verwendung des proteolytisch spaltbaren Helferphagen in Kombination mit der Antigen-spezifischen Elution durch den Liganden oder Teile des Liganden (z.B. Sialyl-Lewis A Antigen) führte zu einer überraschend hohen Varianz an Surrogat-Molekülen in Form von scFv-Fragmenten, die die Bindung des Rezeptors mit dem Ligand inhibieren. Die Liganden und ihre Surrogat-Moleküle können dabei beispielsweise als Agonisten oder Antagonisten für bestimmte biologische Prozesse wirken.

In einer anderen bevorzugten Ausführungsform der Erfindung kann das Verfahren zur Isolierung von Varianzen spezifischer Moleküle dienen, die eine besonders hohe Affinität gegenüber dem Zielmolekül aufweisen. Dies kann beispielsweise dadurch erreicht werden, dass vor der eigentlichen spezifischen Elution mit dem Selektionsmolekül ein Präelutionsschritt gemacht wird, der schwächer bindende spezifische Moleküle eluiert, so dass nur höher oder hochaffine spezifische Moleküle durch die darauffolgende spezifische Elution isoliert werden. Der Präelutionsschritt kann beispielsweise durch eine geringere Konzentration des Selektionsmoleküls oder durch ein anderes Molekül erreicht werden, das das Zielmolekül an der gewünschten Stelle schwächer als das Selektionsmolekül bindet. Alternativ können geringere Konzentrationen des Selektionsmoleküls oder ein anderes Molekül, das das Zielmolekül an der gewünschten Stelle schwächer als das Selektionsmolekül bindet, kompetitiv bei der Bindung der Phagen an das Zielmolekül eingesetzt werden und die spezifische Elution mit dem Selektionsmolekül wie beschrieben erfolgen. Dies wird im Beispiel 3 näher beschrieben, in der größere Varianzen von höher affinen Surrogat-Molekülen für das Lewis Y Antigen in der Form von Antikörperfragmenten, isoliert werden, indem ein Antikörper verwendet wird, der neben Lewis Y eine geringere Affinität gegenüber Lewis B aufweist und Lewis B in der Praeelution und Lewis Y in der Elution verwendet wirde. Dazu werden scFv-pIII(Wildtyp)-Phagemidvektoren und modifizierte pIII-Helferphagen, die durch Einführung einer Trypsin-Spaltungsstelle proteolytisch spaltbar gemacht wurden, eingesetzt. Die Verwendung des proteolytisch spaltbaren Helferphagen in Kombination mit der Antigen-spezifischen Elution (z.B. mit dem Lewis Y Antigen) und einer Präelution mit einer geringeren Menge eines alternativen Antigens (z.B. Lewis B), das vom Antikörper schwächer gebunden wird, führt zu einer überraschend hohen Varianz an höher-affinen Surrogat-Molekülen in Form von scFv-Fragmenten, die die Bindung der Antikörper an das Antigen besonders stark inhibieren.

Gegenstand der vorliegenden Erfindung ist neben dem Selektionsverfahren auch ein Testkit zur Durchführung des erfindungsgemäßen Verfahrens, der die zur Durchführung notwendigen Komponenten enthält. In einer Ausführungsform der Erfindung kann der Testkit die Phagemid-Gen-Bibliothek und einen Helferphagen, der durch einen Inaktivierungsstoff inaktivierbar ist, enthalten. Beispielsweise für die spezielle Ausführungsform der proteolytischen Phageninaktivierung kann der Testkit so einen Helferphagen mit Protease-sensitivem pIII enthalten. Zusätzlich kann der Testkit weitere Komponenten umfassen, wie z.B. das Inaktivierungsagens, vorzugsweise eine oder mehrere Proteasen, besonders bevorzugt Trypsin. Außerdem kann der Testkit zusätzlich ein oder mehrere Zielmoleküle und/oder ein oder mehrere Selektionsmoleküle umfassen.

In einer weiteren Ausführungsform der Erfindung umfasst der Testkit zur Durchführung des erfindungsgemäßen Verfahrens mindestens ein oder mehrere Zielmoleküle und ein oder mehrere Selektionsmoleküle wie sie zur Durchführung des Verfahrens oben beschrieben sind.

Nachfolgend soll die Erfindung an Ausführungsbeispielen näher erläutert werden, aber nicht auf diese beschränkt sein:

### 1. Beispiel

### Generierung von Surrogat-Molekülen auf der Basis von Antikörperfragmenten für die Kohlenhydratstruktur Lewis Y und Vergleich verschiedener Elutionsmethoden

Der LewisY-Antikörper, der das Tetrasaccharid LewisY erkennt, wurde nach Standardmethoden als Zellkulturüberstand aus dem Hybridom A70-C/C8 (IgM, κ) gewonnen und diente in der Selektionsmethode als Zielmolekül zur Generierung von Lewis Y Surrogat-Molekülen.

Für die Untersuchungen wurde eine synthetische Phagemid Antikörperbibliothek verwendet, die nach bekannten Standardmethoden hergestellt wurde (Nissim, A. *et al.* 1994, EMBO J. 13:692-69; Griffiths,A. et al., 1994, EMBO J., 13: 3245-3260; http://www.mrc-cpe.cam.ac.uk/-page/). Die Hauptcharakteristika der Bibliothek sind folgende: sie basiert auf einem einzigen humanen Framework für die schwere Kette V_{H} (V3-23/DP-47 and J_{H}4b) und die leichte Kette V_{L}(O12/O2/DPK9 and J_{L}1), welches die häufigste humane kanonische Struktur darstellt; die CDR3-Region der schweren Kette besitzt die minimale Länge, die zur Bildung einer Antigenbindungsstelle erforderlich ist; in den CDR2- und CDR3-Regionen wurden diejenigen Aminosäuren variiert, die den Kontakt zum Antigen herstellen und im natürlichen, gereiften Repertoire die höchste Diversität aufweisen; der Phagemid-Vektor basiert auf dem bekannten pHEN Vektor; die Bibliothek wurde durch Bindung an Protein-L und Protein-A auf korrekt gefaltete Moleküle vorselektioniert und besitzt eine Diversität von 0,5-2,5 x 10⁸.

Als Helferphage wurde der protease-sensitive Helferphage KM13 verwendet (Kristensen und Winter, 1998). Das Prinzip ist in Figur 1 beschrieben. Es zeigt den Aufbau eines Phagen, der beispielshaft ein Fusionsproteinmolekül des scFv mit dem nicht modifizierten pIII und weitere protease-sensitive modifizierte pIII Moleküle trägt, die vom genetischen Material des Helferphagen kodiert wurden. Während der Verpackung der Phagen einer Phagemid-Bibliothek, die durch die Superinfektion mit Helferphagen induziert wurden, kompetitieren das modifizierte pIII das des Helferphagen (Fig.1, II) mit dem scFv-pIII Fusionsprotein (Fig.1, I) um den Einbau in die Phagenpartikel. KM13 kodiert für ein modifiziertes pIII mit zusätzlichen Proteaseschnittstellen, darunter einer Trypsinschnittstelle zwischen den Domänen D2 und D3 (Fig.1, II). Alle drei Domänen des pIII sind essentiell für die Infektivität des Phagen (Riechmann und Holliger, 1997), wobei es möglich ist, Peptide zwischen die Domänengrenzen einzubauen ohne die Infektivität zu zerstören (Smith, 1985, Krabber etal., 1997). Das wildtyp pIII (pIII(wt)) des scFv-pIII(wt) Fusionsproteins (Fig.1, I) kann durch Trypsin nicht gespalten werden und erhält dadurch die Infektivität des Phagenpartikels nach Trypsinsspaltung. Etwa 99% der produzierten Phagen im Phagemid-System tragen keine scFv und verlieren durch eine Trypsinbehandlung ihre Infektivität. Die Trypsinspaltungsstell im Myc-Tag des scFvpIII(wt) erlaubt eine direkte proteolytische Elution eines infektiösen Phagen vom Antigen, was in der 5. getesteten Elutionsmethode ausgenutzt wurde (s.u.). Die Herstellung und Bestimmung des Titers des Helpherphagen lief nach an sich bekannten Verfahren (Kristensen und Winter, 1998).

Die Phagenproduktion aus der bakteriellen Bibliothek oder aus den späteren Selektionen wurde durch die Superinfektion mit dem Helferphagen KM13 induziert. Dazu wurden Bakterienkulturen in der logarithmischen Wachstumsphase (OD₆₀₀= 0,5-0,6) mit dem Helferphagen im Verhältnis 1:20 ( Anzahl der Bakterien: Anzahl der Phagenpartikel) für 30 min bei 37 °C infiziert und im Selektionsmedium (TY mit 100 µg/ml Ampicillin und 25 µg/ml Kanamycin) bei 30 °C über Nacht kultiviert. Die Phagenpartikel wurden mittels Polyethylenglykol-Fällung aus dem Kulturüberstand gewonnen und in PBS resuspendiert.
Der Phagentiter (Anzahl an Phagenpartikeln/ml) wurde über Verdünnungsreihen der Phagenpräparationen und anschließende Infektion von E. coli TG1 bestimmt. Der Anteil an scFvtragenden Phagenpartikeln wurde durch vergleichende Tiration trypsinierter und nichttrypsinierter Phagenpräparationen ermittelt.
Bei den Selektionen wurde der Zellkulturüberstand des Hybridoms A70-C/C8 an Maus-IgM- spezifischen Magnetpartikeln (Dynal, Hamburg) immobilisiert (6 ml Überstand/200 µl Magnetpartikel, mindestens 2 h bei Raumtemperatur (RT)). Nach einem Waschschritt mit TPBS (PBS/0,1% Tween20) wurden die mit A70-C/C8 gekoppelten Magnetpartikel mit 4% MPBS (4% Magermilchpulver in PBS) für 1h bei RT blockiert. Für die erste Selektionsrunde wurden 5 x 10¹² Phagenpartikel verwendet, die statistisch ca. 500 Kopien jedes scFv der Bibliothek enthalten, und mit den mit A70-C/C6 beladenen Magnetkugel für 2h bei RT inkubiert. Anschließend wurden die Magnetkugeln gewaschen (erste Selektionsrunde: 8 x 2% MPBS + 8 x PBS/0,1% Tween20 + 2 x PBS; weitere Selektionsrunden : 16 x 2% MPBS + 16 x PBS/0,1% Tween20 + 2 x PBS) und die Phagen eluiert.
Für die Elution der Phagen wurden verschiedene Elutionsmethoden vergleichend angewendet:
1. unspezifische Elution
2. unspezifische Elution mit anschließender Trypsinierung
3. spezifische Elution
4. spezifische Elution mit anschließender Trypsinierung
5. Elution durch Trypsinierung (möglich durch eine Trypsin-Spaltstelle im Myc-Tag; Figur 1)
Bei der unspezifische Elution wurde ein 50 mM Glycin-Puffer (pH 2,2; 450 µl) eingesetzt, um die Phagen von den Magnetpartikeln abzulösen (30min bei RT).
Für die spezifische Elution wurde ein PAA-gekoppeltes LewisY-Tetrasaccharid als Antigen verwendet (450 µl, 100 µg/ml in 50 mM Tris, 1 mM CaCl₂ pH 8).
Die Trypsinierung erfolgte bei einer Konzentration von 1 mg/ml in 500µl 50 mM Tris, 1 mM CaCl₂ pH 8 für 15 min bei RT.

Mit den eluierten Phagen wurden 9,5 ml E.coli TG1 in der logarhithmischen Wachstumsphase infiziert (30 min 37 °C), diese dann auf 2xTY-Platten mit Ampicillin und Glucose ausplattiert und durch Verdünnungsreihen der Titer und damit die Menge an eluierten Phagen bestimmt. Für weitere Selektionsrunden wurden die Kolonien von den Platten isoliert und als Glycerindauerkultur (2*TY/ 15-20% Glyzerin) bei -80°C konserviert. Von diesen Dauerkulturen wurden 100 µl für die Anzucht der Bakterien in den nächsten Selektionsrunden verwendet. Einzelkolonien wurden für die weitere Analyse gepickt, Gefrierdauerkulturen angelegt und Phagenüberstände produziert, die dann im ELISA auf spezifische antiidiotypische Bindung analysiert wurden.

Die Phagenüberstände wurden wie in der Phagenpräpäration der Bibliothek beschrieben durch Superinfektion mit KM13 und PEG-Fällung hergestellt. Dies wurde je nach Bedarf in verschiedenen Volumenformaten vorgenommen. Für die ELISA Spezifitätsuntersuchung wurden die Phagen in 200 µl Volumina in 96-Loch-Kulturplatten hergestellt. Die Phagen für die Phageninhibitionsversuche wurden aus 120 ml Überstand mit Hilfe einer wiederholten PEG-Fällung in 2 ml PBS gewonnen und konzentriert.

Für die Spezifiätsuntersuchung im ELISA wurde ein polyklonaler µ-Ketten-spezifischer Ziege-anti-Maus-IgM-Antikörper (anti-IgM) in einer 96-Loch-Platte immobilisiert (240 ng/well, über Nacht bei 4°C, Maxisorb, Nunc), der nach dem Waschen (TPBS) und Blockieren der Platte (4% MPBS) zur Bindung des spezifischen Antikörpers (A70-C/C8-Überstand 1:3 verdünnte Hybridomkulturüberstand) bzw. eines Kontrollantikörpers gleichen Isotyps (TEPC183) führt. Gleiche eingesetzte Mengen wurden über die gleichen Antikörper in gereinigter Form abgeglichen. Anschließend wurde mit den PEG-gefällten scFv-Phagen der einzelnen Klone inkubiert. Die Bindung der scFv-Phagen wurde mit einem monoklonalen Peroxidase-gekoppelten anti-M13-Antikörper (Amersham Pharmacia Biotech, Freiburg) nachgewiesen. Als Substrat wurde 3'3'5'5'-Tetramethylbenzidin verwendet, die Farbreaktion wurde durch Zugabe von 2,5 N Schwefelsäure gestoppt. Die Messung erfolgte bei 450 und 620nm.
Als zusätzliche Kontrollen diente die Bestimmung der Bindung an leere, blockierte Oberflächen (Bindung an Plastik oder Blockierungsmittel) und an den polyklonaler µ-Ketten-spezifischer Ziege-anti-Maus-IgM-Antikörper speziesübergreifender Ig-Binder). Alle Waschvorgänge zwischen den einzelnen Inkubationsschritten wurden mit TPBS (4 x) durchgeführt, mit 2 zusätzlichen Waschgängen mit PBS vor der Substratzugabe.
Als Schwellenwert für die Identifizierung von scFv-Phagen, die den Auswahlkriterien für Surrogatmoleküle für LewisY im ELISA darstellen, wurde eine Bindung an A70-C/C8 mit einer Absorbtion von min. 0,15 (= A₄₅₀ -A₆₂₀) und eine Bindung an die Kontrollantikörper mit weniger als 20% ihrer Bindung an an A70-C/C8 (jeweils nach Abzug der Hintergrundwerte) festgelegt. Für einen Isotyp-Binder musste das Signal für die Bindung an A70-C/C8 und TEPC183 eine OD von 0,15 übersteigen bei einem Hintergrundsignal von <50 %. Generelle Antikörperbinder wurden definiert durch ihre Bindung an A70-C/C8, TEPC183 und anti-IgM, unspezifische Binder durch Bindung ohne immobilisierte Antikörper.

Klone, die den Kriterien eines Surrogatmoleküls entsprachen, Bindung an den LeY-spezifischen Antikörper und keine Bindung an die Kontrollen, wurden anschließend sequenziert. Die Sequenzierung erfolgte mit Standardmethoden, indem die VH und VL Gene der isolierten Phagen durch PCR mit Taq-Polymerase und den Primern CAG GAA ACA GCT ATG AC und GAA TTT TCT GTA TGA GG mit 25 Zyklen aus Balteriengefrierkulturen (15-20% Glyzerin) der Klone amplifiziert, die Produkte mit QIAquick (PCR purification kit, QIAgen) gereinigt und mit Hilfe des Primers CTA TGC GGC CCC ATT CA sequenziert wurden. Die Sequenzierung wurde mit Hilfe von fluoreszenten Dideoxy Ketten Terminatoren (Dye Terminator Sequencing Kit II, Amersham Pharmacia Biotech, Freiburg, Germany) nach Standardmethode durchgeführt (GAG, Bremen).

Klone mit verschiedenen Sequenzen wurde in Inhibitionsanalysen im ELISA getestet, ob sie inhibitorisch als Surrogatmoleküle des Antigens wirken können. Hierzu wurden in 96-Loch-Platten 10µg/ml LewisY-PAA in PBS für 3h bei RT und über Nacht bei 4°C immobilisert. Die Platten wurden gewaschen und mit 4% MPBS für 2 h bei RT blockiert. A70-C/C8 Hybridomkulturüberstand (1:5 und 1:20) wurde mit verschiedenen Mengen an scFv-Phagen (10¹² bis 10¹⁰, von denen etwa 1% scFv tragen) in 2% MPBS/0,1% Tween20 für 1h bei RT in einem blockierten Röhrchen vorinkubiert. Anschließend wurden 50µl/Loch in die mit Antigen beschichteten Platte für 1,5h bei RT überführt. Anschließend wurde 4 mal mit PBS/0,1% Tween20 gewaschen und mit dem Sekundärantikörper (Peroxidase-konjugierter Kaninchen anti-Maus Immunoglobulin, 1:2000) in 2% MPBS/ 0,1% Tween20 für 1h bei RT inkubiert. Die Platten wurden wie im obigen ELISA beschrieben gewaschen und entwickelt.

In Tabelle 1 ist der Effekt der verschiedenen Elutionstechniken vergleichend aufgeführt. Surrogat-scFv sind dabei solche, die nur an den A70-C/C8, aber nicht an die Kontrollen binden. Isotyp-Binder sind solche, die an A70-C/C8 und an Antikörper des gleichen Isotyps (Maus IgM) binden. Generelle Antikörperbinder sind solche, die an alle verwendeten Antikörper binden. Unspezifische Binder binden an das Plattenmaterial oder an das Blockierungsmittel. Nicht-bindende Phagen sind solche, die nicht binden.

Die Tabelle 2 zeigt die Ausbeute an Surrogatmolekülen, gewonnen nach dem erfindungsgemäßen Verfahren nach 2 und 3 Runden der Selektion mit Hilfe der spezifische Elution mit anschließender Trypsinierung, und ihre Diversität, die durch Sequenzierung bestimmt wurde.

Die Ergebnisse zeigen, dass die erfindungsgemäße Selektionsmethode mit kombinierter Elution mit anschließender Trypsinierung den anderen bekannten und getesteten Elutionsmethoden für die Generierung von hohen Varianzen weit überlegen ist.

Erfindungsgemäß wurden 28 Surrogat-Moleküle in Form von scFv-Antikörperfragmenten für LeY aus 96 Klonen nach nur 2 Selektionsrunden isoliert. Eine weitere Selektionsrunde führte zu 76 Surrogat-Molekül-Klonen aus 96 getesteten Klonen. Von diesen Surrogatmolekülen besaßen 65-67 % verschiedene Aminosäuresequenzen, wobei überraschenderweise kein signifikanter Unterschied in der Varianz zwischen der Verwendung von 2 oder 3 Selektionsrunden deutlich wurde. Mit der starken Anreicherung von Phagen mit spezifischen Surrogatmolekülen kam es zu einer Abreicherung unspezifischer scFv-Phagen und solcher scFv-Phagen mit generellen Antikörperbindern. Im Gegenzug dazu reicherten die anderen Elutionsmethoden generelle Antikörperbinder, unspezifischer Binder, und Nicht-Binder an und ergaben keine oder im Fall der spezifischen Elution alleine eine nur minimale, vernachlässigbare Anreicherung für SurogatscFv.
Inhibitionsversuche im ELISA zeigen, dass nahezu alle der isolierten Surrogat-scFv die Bindung des LewisY Antigens an A70-C/C8 spezifisch inhibieren.

### 2. Beispiel

### Generierung von Surrogat-Molekülen auf der Basis von Antikörperfragmenten für den Kohlenhydratliganden von E-Selektin

Die zytoplasmatische Domäne des E-Selektin wurde in rekombinanter Form (aus CHO-Zellen, R+D Systems #ADP-1) in den Versuchen verwendet. E-Selektin bindet in der Gegenwart von Ca2+ die Liganden Sialyl-LewisA und Sialyl-LewisX und spielt in der Zelladhesion beispielsweise von aktivierten Leukozyten an das Endothel ein Rolle.
Für die Untersuchungen wurde eine synthetische Phagemid Antikörperbibliothek verwendet, die nach bekannten Standardmethoden hergestellt wurde (Nissim, A. *et al.* 1994, EMBO J. 13:692-69; Griffiths,A. et al., 1994, EMBO J., 13: 3245-3260; http://www.mrc-cpe.cam.ac.uk/∼phage/). Die Hauptcharakteristika der Bibliothek sind folgende: sie basiert auf einem einzigen humanen Framework für die schwere Kette V_{H} (V3-23/DP-47 and J_{H}4b) und die leichte Kette V_{L}(O12/O2/DPK9 and J_{L}1), welches die häufigste humane kanonische Struktur darstellt; die CDR3-Region der schweren Kette besitzt die minimale Länge, die zur Bildung einer Antigenbindungsstelle erforderlich ist; in den CDR2- und CDR3-Regionen wurden diejenigen Aminosäuren variiert, die den Kontakt zum Antigen herstellen und im natürlichen, gereiften Repertoire die höchste Diversität aufweisen; der Phagemid-Vektor basiert auf dem bekannten pHEN Vektor; die Bibliothek wurde durch Bindung an Protein-L und Protein-A auf korrekt gefaltete Moleküle vorselektioniert und besitzt eine Diversität von 0,5-2,5 x 10⁸.

Als Helferphage wurde der protease-sensitive Helferphage KM13 verwendet (Kristensen und Winter, 1998). Das Prinzip ist in Figur 1 beschrieben. Es zeigt den Aufbau eines Phagen, der beispielshaft ein Fusionsproteinmolekül des scFv mit dem nicht modifizierten pIII und weitere protease-sensitive modifizierte pIII Moleküle trägt, die vom genetischen Material des Helferphagen kodiert wurden. Während der Verpackung der Phagen einer Phagemid-Bibliothek, die durch die Superinfektion mit Helferphagen induziert wurden, kompetitieren das modifizierte pIII das des Helferphagen (Fig.1, II) mit dem scFv-pIII Fusionsprotein (Fig.1, I) um den Einbau in die Phagenpartikel. KM13 kodiert für ein modifiziertes pIII mit zusätzlichen protease Schnittstellen, darunter einer Trypsinschnittstelle zwischen den Domänen D2 und D3 (Fig.1, II). Alle drei Domänen des pIII sind essentiell für die Infektivität des Phagen (Riechmann und Holliger, 1997), wobei es möglich ist, Peptide zwischen die Domänengrenzen einzubauen ohne die Infektivität zu zerstören (Smith, 1985, Krabber etal., 1997). Das wildtyp pIII (pIII(wt)) des scFv-pIII(wt) Fusionsproteins (Fig.1, I) kann durch Trypsin nicht gespalten werden und erhält dadurch die Infektivität des Phagenpartikels nach Trypsinsspaltung. Etwa 99% der produzierten Phagen im Phagemid-System tragen keine scFv und verlieren durch eine Trypsinbehandlung ihre Infektivität. Die Herstellung und Bestimmung des Titers des Helpherphagen lief nach an sich bekannten Verfahren (Kristensen und Winter, 1998).

Die Phagenproduktion aus der bakteriellen Bibliothek oder aus den späteren Selektionen wurde durch die Superinfektion mit dem Helferphagen KM13 induziert. Dazu wurden Bakterienkulturen in der logarithmischen Wachstumsphase (OD₆₀₀= 0,5-0,6) mit dem Helferphagen im Verhältnis 1:20 ( Anzahl der Bakterien: Anzahl der Phagenpartikel) für 30 min bei 37 °C infiziert und im Selektionsmedium (TY mit 100 µg/ml Ampicillin und 25 µg/ml Kanamycin) bei 30 °C über Nacht kultiviert. Die Phagenpartikel wurden mittels Polyethylenglykol-Fällung aus dem Kulturüberstand gewonnen und in PBS resuspendiert.
Der Phagentiter (Anzahl an Phagenpartikeln/ml) wurde über Verdünnungsreihen der Phagenpräparationen und anschließende Infektion von E. coli TG1 bestimmt.

Bei den Selektionen wurde 60 µg des rekombinanten E-Selektins in 2 ml an Immunotubes über Nacht bei 4°C in TC-Puffer (50mM Tris pH 7.4, 10mM CaCl₂) immobilisiert. Nach einem Waschschritt mit TC- Puffer (3x1 Volumen) wurde die Immunotube mit 30%FKS/RPMI/TC sowie die Phagenbibliothek mit 10%FKS/RPMI/TC für 1.5 h h bei RT blockiert. Für die erste Selektionsrunde wurden 5 x 10¹² Phagenpartikel verwendet, die statistisch ca. 500 Kopien jedes scFv der Bibliothek enthalten, der Phagemid-scFv-Bibliothek für 0.5 h bei RT zugegeben. Anschließend wurde die Immunotube gewaschen (erste Selektionsrunde: 4 x 10%FKS/RPMI/TC + 4 x PBS/0,1% Tween20 + 2 x PBS; weitere Selektionsrunden : 5 x 10%FKS/RPMI/TC + 6 x TC/0,1% Tween20 + 4 x TC). Anschließend wurden die Phagen mit PAA-gekoppeltem Sialyl-LewisA-Tetrasaccharid (450 µl, 111 µg/ml in 50 mM Tris, 1 mM CaCl₂ pH 8) spezifisch eluiert und die eluierten Phagen für 15 min bei RT mit Trypsin behandelt (1 mg/ml Trypsin in 500µl 50 mM Tris, 1 mM CaCl₂ pH 8).

Mit den eluierten Phagen wurden 9,5 ml E.coli TG1 in der logarhithmischen Wachstumsphase infiziert (30 min 37 °C), diese dann auf 2xTY-Platten mit Ampicillin und Glucose ausplattiert und durch Verdünnungsreihen der Titer und damit die Menge an eluierten Phagen bestimmt. Für weitere Selektionsrunden wurden die Kolonien von den Platten isoliert und als Glyzerindauerkultur (2*TY/ 15-20% Glyzerin) bei -80°C konserviert. Von diesen Dauerkulturen wurden 100 µl für die Anzucht der Bakterien in den nächsten Selektionsrunden verwendet. Einzelkolonien wurden für die weitere Analyse gepickt, Gefrierdauerkulturen angelegt und Phagenüberstände produziert, die dann im ELISA auf spezifische antiidiotypische Bindung analysiert wurden.

Die Phagenüberstände wurden wie in der Phagenpräpäration der Bibliothek beschrieben durch Superinfektion mit KM13 und PEG-Fällung hergestellt. Dies wurde je nach Bedarf in verschiedenen Volumenformaten vorgenommen. Für die ELISA Spezifitätsuntersuchung wurden die Phagen in 200 µl Volumina in 96-Loch-Kulturplatten hergestellt. Die Phagen für die Phageninhibitionsversuche wurden aus 120 ml Überstand mit Hilfe einer wiederholten PEG-Fällung in 2 ml PBS gewonnen und konzentriert.

Für die Spezifiätsuntersuchung im ELISA wurde E-Selektin in TC-Puffer (50mM Tris pH 7.4, 10mM CaCl₂) in einer 96-Loch-Platte immobilisiert (200 ng/well, über Nacht bei 4°C, pvc falcon), der nach dem Waschen (TC) und Blockieren der Platte (30%FKS/RPMI/TC) zur Bindung des spezifischen Phagen benutzt. Anschließend wurde mit mit den PEG-gefällten scFv-Phagen (ca. 10¹²/ml) der einzelnen Klone in inkubiert. Die Bindung der scFv-Phagen wurde mit einem monoklonalen Peroxidase-gekoppelten anti-M13-Antikörper (Amersham Pharmacia Biotech, Freiburg) nachgewiesen. Als Substrat wurde 3'3'5'5'-Tetramethylbenzidin verwendet, die Farbreaktion wurde durch Zugabe von 2,5 N Schwefelsäure gestoppt. Die Messung erfolgte bei 450 und 620nm.
Als zusätzliche Kontrollen diente die Bestimmung der Bindung an leere, blockierte Oberflächen (Bindung an Plastik oder Blockierungsmittel). Alle Waschvorgänge zwischen den einzelnen Inkubationsschritten wurden mit TC/0.1%Tween (4 x) durchgeführt, mit 2 zusätzlichen Waschgängen mit TC vor der Substratzugabe.
Als Schwellenwert für die Identifizierung von scFv-Phagen, die den Auswahlkriterien für Surrogatmoleküle für Sialyl-LewisA im ELISA darstellen, wurde eine Bindung an E-Selektin mit einer Absorbtion von min. 0,15 (= A₄₅₀ -A₆₂₀) und eine Bindung in den Kontroll-wells mit weniger als 20% ihrer Bindung an an E-Selektin (jeweils nach Abzug der Hintergrundwerte) festgelegt.

Klone, die den Kriterien eines Surrogatmoleküls für den E-Selektinliganden entsprachen, wurden anschließend sequenziert. Die Sequenzierung erfolgte mit Standardmethoden, indem die VH und VL Gene der isolierten Phagen durch PCR mit Taq-Polymerase und den Primern CAG GAA ACA GCT ATG AC und GAA TTT TCT GTA TGA GG mit 25 Zyklen aus Balteriengefrierkulturen (15-20% Glyzerin) der Klone amplifiziert, die Produkte mit QIAquick (PCR purification kit, QIAgen) gereinigt und mit Hilfe des Primers CTA TGC GGC CCC ATT CA sequenziert wurden. Die Sequenzierung wurde mit Hilfe von fluoreszenten Dideoxy Ketten Terminatoren (Dye Terminator Sequencing Kit II, Amersham Pharmacia Biotech, Freiburg, Germany) nach Standardmethode durchgeführt (GAG, Bremen).

Klone mit verschiedenen Sequenzen wurden in Inhibitionsanalysen im ELISA getestet, ob sie inhibitorisch als Surrogatmoleküle des Antigens wirken können. Hierzu wurde E-Selektin in TC-Puffer (50mM Tris pH 7.4, 10mM CaCl₂) in einer 96-Loch-Platte immobilisiert (200 ng/well, über Nacht bei 4°C, pvc falcon), der nach dem Waschen (TC) und Blockieren der Platte (30%FKS/RPMI/TC) zur Bindung des spezifischen Zuckers (Sialyl-LewisA-PAA-Biotin bzw. Sialyl-LewisA-PAA benutzt. Anschließend wurde mit den PEG-gefällten scFv-Phagen der einzelnen Klone in verschiedenen Konzentrationen von 0 bis ca 10¹¹ Phagen/well inkubiert. Die Bindung der scFv-Phagen bzw. des Sialyl-LewisA-PAA-Biotin wurde mit einem monoklonalen Peroxidase-gekoppelten anti-M13-Antikörper (Amersham Pharmacia Biotech, Freiburg) bzw mit Peroxidase gekoppeltem Strptavidin nachgewiesen. Als Substrat wurde 3'3'5'5'-Tetramethylbenzidin verwendet, die Farbreaktion wurde durch Zugabe von 2,5 N Schwefelsäure gestoppt. Die Messung erfolgte bei 450 und 620nm. Die Platten wurden wie im obigen ELISA beschrieben gewaschen und entwickelt.

Tabelle 3 zeigt die Ausbeute an Surrogatmolekülen, gewonnen nach 2 Runden dererfindungsgemäßen Selektion mit Hilfe der spezifische Elution mit anschließender Trypsinierung, und ihre Diversität, die durch Sequenzierung bestimmt wurde.

Die Ergebnisse zeigen, dass die erfindungsgemäßen Selektionsmethode mit der spezifischen Elution kombiniert mit anschließender Trypsinierung eine hohe Varianz an Surrogat-Liganden für den E-Selektin-Kohlenhydratliganden ergibt.

Inhibitionsversuche im ELISA zeigen, dass nahezu alle der isolierten Surrogat-scFv die Bindung des Sialyl-LewisA Antigens an das rekombinante E-Selektin spezifisch inhibieren.

### 3. Beispiel

### Generierung von Surrogat-Molekülen hoher Affinität auf der Basis von Antikörperfragmenten für die Kohlenhydratstruktur LewisY

Der Antikörper A70-A/A9 erkennt der das Tetrasaccharid LewisY und hat eine Kreuzreaktivität mit dem Tetrasaccharid LewisB, die ca. um das 20-100-fache schwächer ist als die Bindung an LewisY. Der Mausantikörper wurde nach Standardmethoden als Zellkulturüberstand aus dem Hybridom A70-A/A9 (IgG1, κ) gewonnen und diente in der Selektionsmethode als Zielmolekül zur Generierung von Lewis Y Surrogat-Molekülen.

Für die Untersuchungen wurde eine synthetische Phagemid Antikörperbibliothek verwendet, die nach bekannten Standardmethoden hergestellt wurde (Nissim, A. *et al.* 1994, EMBO J. 13:692-69; Griffiths,A. et al., 1994, EMBO J., 13: 3245-3260; http://www.mrc-cpe.cam.ac.uk/∼phage/). Die Hauptcharakteristika der Bibliothek sind folgende: sie basiert auf einem einzigen humanen Framework für die schwere Kette V_{H} (V3-23/DP-47 and J_{H}4b) und die leichte Kette V_{L}(O12/O2/DPK9 and J_{L}1), welches die häufigste humane kanonische Struktur darstellt; die CDR3-Region der schweren Kette besitzt die minimale Länge, die zur Bildung einer Antigenbindungsstelle erforderlich ist; in den CDR2- und CDR3-Regionen wurden diejenigen Aminosäuren variiert, die den Kontakt zum Antigen herstellen und im natürlichen, gereiften Repertoire die höchste Diversität aufweisen; der Phagemid-Vektor basiert auf dem bekannten pHEN Vektor; die Bibliothek wurde durch Bindung an Protein-L und Protein-A auf korrekt gefaltete Moleküle vorselektioniert und besitzt eine Diversität von 0,5-2,5 x 10⁸.

Als Helferphage wurde der protease-sensitive Helferphage KM13 verwendet (Kristensen und Winter, 1998). Das Prinzip ist in Figur 1 beschrieben. Es zeigt den Aufbau eines Phagen, der beispielshaft ein Fusionsproteinmolekül des scFv mit dem nicht modifizierten pIII und weitere protease-sensitive modifizierte pIII Moleküle trägt, die vom genetischen Material des Helferphagen kodiert wurden. Während der Verpackung der Phagen einer Phagemid-Bibliothek, die durch die Superinfektion mit Helferphagen induziert wurden, kompetitieren das modifizierte pIII das des Helferphagen (Fig.1, II) mit dem scFv-pIII Fusionsprotein (Fig.1, I) um den Einbau in die Phagenpartikel. KM13 kodiert für ein modifiziertes pIII mit zusätzlichn protease Schnittstellen, darunter einer Trypsinschnittstelle zwischen den Domänen D2 und D3 (Fig.1, II). Alle drei Domänen des pIII sind essentiell für die Infektivität des Phagen (Riechmann und Holliger, 1997), wobei es möglich ist, Peptide zwischen die Domänengrenzen einzubauen ohne die Infektivität zu zerstören (Smith, 1985, Krabber etal., 1997). Das wildtyp pIII (pIII(wt)) des scFv-pIII(wt) Fusionsproteins (Fig.1, I) kann durch Trypsin nicht gespalten werden und erhält dadurch die Infektivität des Phagenpartikels nach Trypsinsspaltung. Etwa 99% der produzierten Phagen im Phagemid-System tragen keine scFv und verlieren durch eine Trypsinbehandlung ihre Infektivität. Die Trypsinspaltungsstell im Myc-Tag des scFvpIII(wt) erlaubt eine direkte proteolytische Elution eines infektiösen Phagen vom Antigen, was in der 5. getesteten Elutionsmethode ausgenutzt wurde (s.u.). Die Herstellung und Bestimmung des Titers des Helpherphagen lief nach an sich bekannten Verfahren (Kristensen und Winter, 1998).

Die Phagenproduktion aus der bakteriellen Bibliothek oder aus den späteren Selektionen wurde durch die Superinfektion mit dem Helferphagen KM13 induziert. Dazu wurden Bakterienkulturen in der logarithmischen Wachstumsphase (OD₆₀₀= 0,5-0,6) mit dem Helferphagen im Verhältnis 1:20 ( Anzahl der Bakterien: Anzahl der Phagenpartikel) für 30 min bei 37 °C infiziert und im Selektionsmedium (TY mit 100 µg/ml Ampicillin und 25 µg/ml Kanamycin) bei 30 °C über Nacht kultiviert. Die Phagenpartikel wurden mittels Polyethylenglykol-Fällung aus dem Kulturüberstand gewonnen und in PBS resuspendiert.
Der Phagentiter (Anzahl an Phagenpartikeln/ml) wurde über Verdünnungsreihen der Phagenpräparationen und anschließende Infektion von E. coli TG1 bestimmt. Der Anteil an scFvtragenden Phagenpartikeln wurde durch vergleichende Tiration trypsinierter und nichttrypsinierter Phagenpräparationen ermittelt.
Bei den Selektionen wurde der Zellkulturüberstand des Hybridoms A70-A/A9 an Maus-IgG-spezifischen Magnetpartikeln (Dynal, Hamburg) immobilisiert (6 ml Überstand/200 µl Magnetpartikel, mindestens 2 h bei Raumtemperatur (RT)). Nach einem Waschschritt mit TPBS ( PBS/0,1% Tween20) wurden die mit A70-A/A9 gekoppelten Magnetpartikel mit 4% MPBS (4% Magermilchpulver in PBS) für 1h bei RT blockiert. Für die erste Selektionsrunde wurden 5 x 10¹² Phagenpartikel verwendet, die statistisch ca. 500 Kopien jedes scFv der Bibliothek enthalten, und mit den mit A70-A/A9 beladenen Magnetkugel für 2h bei RT inkubiert. Anschließend wurden die Magnetkugeln gewaschen (erste Selektionsrunde: 8 x 2% MPBS + 8 x PBS/0,1% Tween20 + 2 x PBS; weitere Selektionsrunden : 16 x 2% MPBS + 16 x PBS/0,1% Tween20 + 2 x PBS). Anschließend wurden in einem Präelutionsschritt die Phagen mit PAA-gekoppeltem LewisB-Tetrasaccharid (450 µl, 5 µg/ml in 50 mM Tris, 1 mM CaCl₂ pH 8) eluiert. Anschließend wurde 2* mit PBS gewaschen und die eigentliche Elution mit PAA-gekoppeltem LewisY-Tetrasaccharid (450 µl, 100 µg/ml in 50 mM Tris, 1 mM CaCl₂ pH 8) durchgeführt. Die eluierten und präeluierten Phagen wurden separat mit Trypsin behandelt (1 mg/ml Trypsin in 500µl 50 mM Tris, 1 mM CaCl₂ pH 8 für 15 min bei RT).

Mit den eluierten und präeluierten Phagen wurden jeweils 9,5 ml E.coli TG1 in der logarhithmischen Wachstumsphase infiziert (30 min 37 °C), diese dann auf 2xTY-Platten mit Ampicillin und Glucose ausplattiert und durch Verdünnungsreihen der Titer und damit die Menge an eluierten Phagen bestimmt. Für weitere Selektionsrunden wurden die Kolonien von den Platten isoliert und als Glycerindauerkultur (2*TY/ 15-20% Glyzerin) bei -80°C konserviert. Von diesen Dauerkulturen wurden 100 µl für die Anzucht der Bakterien in den nächsten Selektionsrunden verwendet. Einzelkolonien wurden für die weitere Analyse gepickt, Gefrierdauerkulturen angelegt und Phagenüberstände produziert, die dann im ELISA auf spezifische antiidiotypische Bindung analysiert wurden.

Die Phagenüberstände wurden wie in der Phagenpräpäration der Bibliothek beschrieben durch Superinfektion mit KM13 und PEG-Fällung hergestellt. Dies wurde je nach Bedarf in verschiedenen Volumenformaten vorgenommen. Für die ELISA Spezifitätsuntersuchung wurden die Phagen in 200 µl Volumina in 96-Loch-Kulturplatten hergestellt. Die Phagen für die vergleichenden relativen Affinitätsversuche wurden aus 40 ml Überstand mit Hilfe einer PEG-Fällung in 2 ml PBS gewonnen und konzentriert.

Für die Spezifiätsuntersuchung im ELISA wurde ein polyklonaler γ-Ketten-spezifischer Ziege-anti-Maus-IgG-Antikörper (anti-IgG) in einer 96-Loch-Platte immobilisiert (240 ng/well, über Nacht bei 4°C, Maxisorb, Nunc), der nach dem Waschen (TPBS) und Blockieren der Platte (4% MPBS) zur Bindung des spezifischen Antikörpers (A70-A/A9-Überstand 1:3 verdünnte Hybridomkulturüberstand) bzw. eines Kontrollantikörpers gleichen Isotyps (MOPC-21) führt. Gleiche eingesetzte Mengen wurden über die gleichen Antikörper in gereinigter Form abgeglichen. Anschließend wurde mit den PEG-gefällten scFv-Phagen der einzelnen Klone inkubiert. Die Bindung der scFv-Phagen wurde mit einem monoklonalen Peroxidase-gekoppelten anti-M13-Antikörper (Amersham Pharmacia Biotech, Freiburg) nachgewiesen. Als Substrat wurde 3'3'5'5'-Tetramethylbenzidin verwendet, die Farbreaktion wurde durch Zugabe von 2,5 N Schwefelsäure gestoppt. Die Messung erfolgte bei 450 und 620nm.
Als zusätzliche Kontrollen diente die Bestimmung der Bindung an leere, blockierte Oberflächen (Bindung an Plastik oder Blockierungsmittel) und an den polyklonaler µ-Ketten-spezifischer Ziege-anti-Maus-IgM-Antikörper speziesübergreifender Ig-Binder). Alle Waschvorgänge zwischen den einzelnen Inkubationsschritten wurden mit TPBS (4 x) durchgeführt, mit 2 zusätzlichen Waschgängen mit PBS vor der Substratzugabe.
Als Schwellenwert für die Identifizierung von scFv-Phagen, die den Auswahlkriterien für Surrogatmoleküle für LewisY im ELISA darstellen, wurde eine Bindung an A70-A/A9 mit einer Absorbtion von min. 0,15 (= A₄₅₀ -A₆₂₀) und eine Bindung an die Kontrollantikörper mit weniger als 20% ihrer Bindung an an A70-A/A9 (jeweils nach Abzug der Hintergrundwerte) festgelegt. Für einen Isotyp-Binder musste das Signal für die Bindung an A70-A/A9 und MOPC-21 eine OD von 0,15 übersteigen bei einem Hintergrundsignal von <50 %. Generelle Antikörperbinder wurden definiert durch ihre Bindung an A70-A/A9, MOPC-21 und anti-IgG, unspezifische Binder durch Bindung ohne immobilisierte Antikörper.

Phagenpräparationen der Klone, die den Kriterien eines Surrogatmoleküls entsprachen, Bindung an den A70-A/A9 und keine Bindung an die Kontrollen, wurden auf ihre relative Affinität zu A70-A/A9 getestet. Die Titer der hierzu PEG-gefällten und konzentrierten Phagen wurden bestimmt und gleiche Phagenkonzentrationen vergleichend in Verdünnungsreihen im ELISA wie oben beschrieben mit A70-A/A9 getestet.

Klone, die den Kriterien eines Surrogatmoleküls entsprachen wurden anschließend sequenziert. Die Sequenzierung erfolgte mit Standardmethoden, indem die VH und VL Gene der isolierten Phagen durch PCR mit Taq-Polymerase und den Primern CAG GAA ACA GCT ATG AC und GAA TTT TCT GTA TGA GG mit 25 Zyklen aus Balteriengefrierkulturen (15-20% Glyzerin) der Klone amplifiziert, die Produkte mit QIAquick (PCR purification kit, QIAgen) gereinigt und mit Hilfe des Primers CTA TGC GGC CCC ATT CA sequenziert wurden. Die Sequenzierung wurde mit Hilfe von fluoreszenten Dideoxy Ketten Terminatoren (Dye Terminator Sequencing Kit II, Amersham Pharmacia Biotech, Freiburg, Germany) nach Standardmethode durchgeführt (GAG, Bremen).

Tabelle 4 zeigt, dass sowohl mit der Präelution, als auch mit der darauffolgenden Elution annähernd vergleichbare Mengen an Phagen nach der 2. Selektionsrunde eluiert wurden, die beide eine hohe Varianz besitzen.

Der Vergleich, von jeweils 8 Klonen aus der Präelution und der Elution nach der 2. Selektionsrunde zeigt, dass die isolierten Surrogat-Moleküle aus der Elution eine deutliche bis um das vielfach höhere Affinität zu A70-A/A9 besitzen als die Surrogat-Moleküle aus der Präelution.

**Tabelle 1**

| | Unspezifische Elution | | Unspezifische Elution mit anschliessender Trypsinbehandlung | | Spezifische Elution | | spezifische Elution mit anschliessender Trypsinbehandlung | | Elution durch Trypsinbehandlung | |
|---|---|---|---|---|---|---|---|---|---|---|
| Selectionsrun den | 1. | 2. | 2. | 1. | 1. | 2. | 1. | 2. | 1. | 2. |
| Koloniezeahl gesamt | 2x10⁶ | 6x10⁵ | 9,4x10⁴ | 6x10⁵ | 6x10⁴ | 2,5x10³ | 1,3x10³ | 1,2x10³ | 2,6x10³ | 2,7x10⁴ |

| Anzahl der bindenden Klone von 96 getesteten Klonen im ELISA | | | | | | | | | | |
|---|---|---|---|---|---|---|---|---|---|---|
| Surrogat-scFv | 5 | 1 | 1 | 0 | 0 | 2 | 3 | 28 | 9 | 1 |
| Isotyp Binder 1 | 1 | 0 | 1 | 0 | 0 | 0 | 0 | 1 | 0 | 0 |
| Generelle Antikörperbinder ² | 33 | 15 | 35 | 29 | 25 | 16 | 33 | 24 | 28 | 49 |
| unspecifische Binder³ | 2 | 3 | 0 | 3 | 3 | 5 | 1 | 1 | 1 | 0 |
| Nicht bindende klone ⁴ | 55 | 77 | 59 | 64 | 68 | 73 | 59 | 42 | 58 | 46 |

**Tabelle 2**

| | A 70-C/C8 | |
|---|---|---|
| Selektionsmolekül | Le^{Y}-PAA [100µg/ml] | |
| Selektionsrunden | 2 | 3 |
| Selektion mit | Magnetkugeln | Magnetkugeln |

| Anzahl der bindenden Klone von 96 getesteten klonen im ELISA | | |
|---|---|---|
| Surrogat-scFv | 28 | 76 |
| Isotyp Binder | 1 | 0 |
| Unspezifische Binder | 1 | 0 |
| Nicht bindende Klone | 42 | 11 |

| Diversität | | |
|---|---|---|
| Anzahl sequenzierter Klone | 23 | 18 |
| Anzahl unterschiedlicher Sequenzen | 15 | 12 |
| Diversität in % | 65 | 67 |

**Tabelle 3**

| Zielmolekül | E-Selektin |
|---|---|
| Selcktionsmolekül | Sialyl-Le^{A}-PAA [111µg/ml] |
| Selektionsrunden | 2 |
| Selektion mit | Magnetkugeln |

| Anzahl der bindenden Klone von 96 getesteten klonen im ELISA | |
|---|---|
| Surrogat-scFv | 37 |
| Unspezifische Binder | 8 |
| Nicht bindende Klone | 51 |
| Anzahl sequenzierter Klone | 12 |
| Anzahl unterschiedlicher Sequenzen | 9¹⁰ |
| Diversität in % | 75% |

**Tabelle 4**

| Zielmolekül A70-A/A9 | | |
|---|---|---|
| Spezifische Elution mit anschliessender Trypsinbehandlung | | |
| | Präelution | Elution |
| Selektionsmolekül | Le^{b}-PAA [5µg/ml] | Le^{Y}-PAA [100µg/ml] |
| Selektionsrunde | 2 | 2 |
| Koloniezahl gesamt | 2,3x10³ | 9x10² |

| Anzahl der bindenden Klone von 96 getesteten Klone im ELISA | | |
|---|---|---|
| Surrogat - scFv | 39 | 32 |
| Isotyp Binder | 0 | 1 |
| Unspezifische Binder | 1 | 1 |
| Nicht bindende klone | 27 | 44 |

| Diversität | | |
|---|---|---|
| Anzahl sequenzierter Klone | 16 | 16 |
| Anzahl unterschiedlicher Sequenzen | 12 | 9 |
| Diversität in % | 75 | 56 |

## Patentansprüche

1. Verfahren zur Isolierung großer Varianzen spezifischer Moleküle für ein Zielmolekül aus Phagemid-Gen-Bibliotheken, indem von einer Phagemid-Gen-Bibliothek ausgegangen wird, die große Varianzen eines Moleküls oder große Varianzen von Teilen eines Moleküls, das als spezifisches Molekül fungiert, direkt oder über einen Linker kovalent an das Phagenhüllprotein pIII des filamentösen Phagen M13 gekoppelt als Fusionsprotein auf der Oberfläche von Phagen enthalten, die Phagen der Bibliothek, die die Fusionsprotein exprimierenden Phagen enthalten, mit einem Zielmolekül inkubiert werden, die spezifisch über das Fusionsprotein an das Zielmolekül gebundenen Phagen mit einem Selektionsmolekül, das die Bindung zwischen dem spezifischen Molekül und dem Zielmolekül aufhebt und an das Zielmolekül bindet, eluiert werden, die eluierten Phagen, die das Fusionsprotein nicht exprimieren, derart inaktiviert werden, dass sie keine Bakterien mehr infizieren können, mit den eluierten Phagen, die das Fusionsprotein exprimieren, Baktieren infiziert werden und die Varianzen des spezifischen Moleküls aus den infizierten Klonen bestimmt und gewonnen werden.

2. Verfahren nach Anspruch 1,
**dadurch gekennzeichnet, dass**
als spezifische Moleküle Trägerproteine mit ein oder mehreren variablen Anteilen oder Antikörperfragmente, vorzugsweise Fab-Fragmente, single domain Fragmente oder scFv-Fragmente isoliert werden.

3. Verfahren nach Anspruch 1,
**dadurch gekennzeichnet, dass**
als Zielmoleküle solche eingesetzt werden, die Rezeptoren oder als Rezeptor wirkende Teile davon für das Selektionsmolekül sind und als Selektionsmoleküle solche eingesetzt werden, die Liganden oder als Liganden wirkende Teile davon für das Zielmolekül sind.

4. Verfahren nach Anspruch 1,
**dadurch gekennzeichnet, dass**
als Zielmoleküle Antikörper oder deren Fragmente eingesetzt werden, die an das Selektionsmolekül spezifisch binden und als Selektionsmoleküle die Antigene oder Teile davon dienen.

5. Verfahren nach Anspruch 1,
**dadurch gekennzeichnet, dass**
als Zielmolekül Lektine eingesetzt werden, die an das Selektionsmolekül spezifisch binden und als Selektionsmolekül Kohlenhydratstrukturen oder deren Teile dienen.

6. Verfahren nach Anspruch 1,
**dadurch gekennzeichnet, dass**
als spezifische Moleküle solche isoliert werden, die Surrogatmoleküle für das Selektionsmolekül oder Teile des Selektionsmoleküls sind.

7. Verfahren nach Anspruch 6,
**dadurch gekennzeichnet, dass**
als Surrogatmoleküle Antikörperfragmente oder Trägerproteine isoliert werden, als Zielmolekül ein Rezeptor oder ein Teil eines Rezeptors dient, der an den als Selektionsmolekül dienenden Liganden oder einen Teil des Liganden bindet.

8. Verfahren nach Anspruch 6,
**dadurch gekennzeichnet, dass**
als Surrogatmoleküle Antikörperfragmente isoliert werden, als Zielmolekül ein Antikörper oder ein Antikörperfragment dient, der/das gegen das als Selektionsmolekül dienende Antigen oder Teile davon gerichtet ist.

9. Verfahren nach Anspruch 1,
**dadurch gekennzeichnet, dass**
die Inaktivierung der Phagen, die das Fusionsprotein und damit das spezifische Molekül nicht exprimieren, proteolytisch unter Verwendung eines Helferphagen mit Protease-sensitivem pIII vorgenommen wird.

10. Verfahren nach Anspruch 1 zur Isolierung großer Varianzen von Surrogat Molekülen für Antigene in Form von single-domain- oder scFv-Antikörperfragmenten, die gegen Antikörper gerichtet sind, die ein tumorassoziiertes Antigen erkennen,
**dadurch gekennzeichnet, dass**
als Phagemid-Gen-Bibliothek eine solche eingesetzt wird, die Antikörperfragment-pIII(Wildtyp)-Phagemidvektoren enthält, als Zielmolekül Antikörper oder Antikörperfragmente gegen das tumorassoziierte Antigen eingesetzt werden, als Selektionsmolekül das tumorassoziierte Antigen oder Teile davon dienen und die Inaktivierung der Phagen, die das Fusionsprotein nicht exprimieren, proteolytisch unter Verwendung eines pIII-Helferphagen, der durch Modifizierung proteolytisch spaltbar ist, erfolgt.

11. Verfahren nach Anspruch 1 zur Isolierung großer Varianzen von Surrogat Molekülen für Liganden in Form von single-domain- oder scFv-Antikörperfragmenten, die gegen Rezeptoren gerichtet sind, die von einem Liganden gebunden werden, **dadurch gekennzeichnet, dass** als Phagemid-Gen-Bibliothek eine solche eingesetzt wird, die Antikörperfragment-pIII(Wildtyp)-Phagemidvektoren enthält, als Zielmolekül der Rezeptor oder Teile des Rezeptors eingesetzt werden, als Selektionsmolekül der Ligand oder Teile des Liganden dienen und die Inaktivierung der Phagen, die das Fusionsprotein nicht exprimieren, proteolytisch unter Verwendung eines pIII-Helferphagen, der durch Modifizierung proteolytisch spaltbar ist, erfolgt.

12. Verfahren nach Anspruch 1,
**dadurch gekennzeichnet, dass**
Varianzen gegenüber dem Zielmolekül höher affiner spezifischer Moleküle gewonnen werden, indem vor der spezifischen Elution mit dem Selektionsmolekül ein weiterer Schritt durchgeführt wird, der entweder eine Präelution ist, in der mit schwächer bindenden Molekülen oder mit einer geringeren Konzentration des Selektionsmoleküls oder einer Kombination beider, schwächer bindende spezifische Moleküle präeluiert werden.

13. Verfahren nach Anspruch 1,
**dadurch gekennzeichnet, dass**
Varianzen gegenüber dem Zielmolekül höher affiner spezifischer Moleküle gewonnen werden durch eine kompetitive Inkubation der die spezifischen Moleküle tragenden Phagen mit dem Zielmolekül zusammen mit einer geringeren Konzentration des Selektionsmolekül oder eines anderen Moleküls, das das Zielmolekül schwächer als das Selektionsmolekül spezifisch bindet, oder einer Kombination beider.

14. Verfahren nach Anspruch 1,
**dadurch gekennzeichnet, dass**,
die Inaktivierung der Phagen, die das Fusionsprotein nicht exprimieren, vor der Inkubation mit dem Zielmolekül und der spezifischen Elution mit dem Selektionsmolekül vorgenommen wird.

15. Testkit zur Isolierung großer Varianzen spezifischer Moleküle für ein Zielmolekül aus Phagemid-Gen-Bibliotheken gemäß dem Verfahren der Ansprüche 1 bis 14, umfassend eine Phagemid-Gen-Bibliothek und einen inaktivierbaren Helferphagen.

16. Testkit nach Anspruch 15,
**dadurch gekennzeichnet, dass**
er einen Helferphagen mit Protease-sensitivem pIII umfasst.

17. Testkit nach Anspruch 15 oder 16,
**dadurch gekennzeichnet, dass**
er zusätzlich das Agens zur Inaktivierung des Helferphagen umfasst, vorzugsweise eine oder mehrere Proteasen.

18. Testkit nach einem der Ansprüche 15 bis 17,
**dadurch gekennzeichnet, dass**
er ein oder mehrere Zielmoleküle und/oder ein oder mehrere Selektionsmoleküle umfasst.

19. Testkit zur Isolierung großer Varianzen spezifischer Moleküle für ein Zielmolekül aus Phagemid-Gen-Bibliotheken gemäß dem Verfahren der Ansprüche 1 bis 14, umfassend ein oder mehrere Zielmoleküle und ein oder mehrere Selektionsmoleküle.
